# EUROPEAN PATENT APPLICATION

(11) **EP 1 052 288 A1**
(43) Date of publication of application: **15.11.2000**
(21) Application number: 99401155.9
(22) Date of filing: 10.05.1999
(51) Int. Cl.: C12N 15/87, C12N 15/88, A61K 48/00, C07K 14/47, C07K 7/08, C07K 16/44

(54) **Complex for transferring an anionic substance of interest into a cell**

(71) Applicant: TRANSGENE S.A., 67082 Strasbourg Cédex (FR)
(72) Inventor: Jacob, Eric, 67140 Stolzheim (FR)
(74) Representative: VOSSIUS & PARTNER

(57) **Abstract**

A complex for transfering an anionic substance of interest into a cell is disclosed that comprises:
(i) at least a first polypeptide capable of binding to an anionic substance,
(ii) a anionic substance of interest,
wherein said first polypeptide comprises all or part of an amino acid sequence selected from the group consisting of the subunit amino acid sequences of the C1 complement factor.

## Description

The present invention concerns complexes comprising all or part of the amino acid sequence of the C1 complement factor and an anionic substance of interest, such as nucleic acids. These complexes are useful for transferring said anionic substance into a cell.

Gene therapy has generally been conceived as principally applicable to heritable deficiency diseases (cystic fibrosis, dystrophies, haemophilias, etc) where permanent cure may be effected by introducing a functional gene. However, a much larger group of diseases, notably acquired diseases (cancer, AIDS, multiple sclerosis, etc) might be treatable by transiently engineering host cells to produce beneficial proteins.

Another application of gene therapy is vaccination. In this regard, all or part of the immunogenic product encoded by the nucleic acid introduced into cells of a vertebrate may be expressed and released or be presented by said cells in the context of the major histocompatibility antigens, thereby eliciting an immune response against the expressed immunogen. Functional nucleic acid can be introduced into cells by a variety of techniques resulting in either transient expression of the gene of interest, referred to as transient transfection, or permanent transformation of the host cells resulting from incorporation of the nucleic acid into the host genome.

Successful gene therapy depends on the efficient delivery to and expression of genetic information within the cells of a living organism. Most delivery mechanisms used to date involve viral vectors, especially adeno- and retroviral vectors. Viruses have developed diverse and highly sophisticated mechanisms to achieve this goal including crossing of the cellular membrane, escape from endosomes and lysosomal degradation, and finally delivery of their genome to the nucleus followed by expression of the viral genome. In consequence, viruses have been used in many gene delivery applications in vaccination or gene therapy applied to humans (see Robbins et al., 1998, Tibtech, 16, 35-40 for a review). The use of viruses suffers from a number of disadvantages: retroviral vectors can not accomodate large-sized DNA (e.g. the dystrophin gene, around 13 kb), the retroviral genome is integrated into host cell DNA and may thus may cause genetic changes in the recipient cell and infectious viral particles could disseminate within the organism or into the environment; adenoviral vectors can induce a strong immune response in treated patients (Mc Coy et al, 1995, Human Gene Therapy, 6, 1553-1560; Yang et al., 1996, Immunity, 1, 433-442). Nevertheless, despite these drawbacks, viral vectors are currently the most useful delivery systems because of their efficiency.

Interestingly, in 1990, Wolff et al. (Science, 247, 1465-1468) have shown that injection of naked RNA or DNA, without any special delivery system, directly into mouse skeletal muscle results in expression of reporter genes within the muscle cells. Nevertheless, although these results indicate that nucleic acid by itself is capable of crossing the plasma membrane of certain cells *in vivo,* the efficiency of the transfection observed in most of the cell types remains very limited due, in particular, to the polyanionic nature of nucleic acids which limits their passage through negatively-charged cell membranes.

Moreover, in 1989, Felgner et al. (Nature, 337, 387-388) already proposed the use of cationic lipids in order to facilitate the introduction of large anionic molecules such as nucleic acids into cells. These cationic lipids are capable of forming complexes with anionic molecules, thus tending to neutralize the negative charges of these molecules allowing to compact the complex, and favoring its introduction into the cell. In this way, related non-viral delivery systems have been developed (see Rolland, 1998, Therapeutic Drug Carrier Systems, 15, 143-198 for a review) which are based on receptor-mediated mechanisms (Perales et al., 1994, Eur. J. Biochem. 226, 255-266; Wagner et al., 1994, Advanced Drug Delivery Reviews, 14, 113-135), on polymer-mediated transfection such as polyamidoamine (Haensler et Szoka, 1993, Bioconjugate Chem., 4, 372-379), dendritic polymer (WO 95/24221), polyethylene imine or polypropylene imine (WO 96/02655), polylysine (US-A- 5 595 897 or FR 2 719 316) or on lipid-mediated transfection (Felgner et al., 1989, Nature, 337, 387-388) such as DOTMA (Felgner et al., 1987, PNAS, 84, 7413-7417), DOGS or Transfectam™ (Behr et al.,1989, PNAS, 86, 6982-6986), DMRIE or DORIE (Felgner et al., 1993, Methods 5, 67-75), DC-CHOL (Gao et Huang, 1991, BBRC, 179, 280-285), DOTAP™ (McLachlan et al., 1995, Gene Therapy, 2,674-622) or Lipofectamine™. These systems present potential advantages with respect to large-scale production, safety, targeting of transfectable cells, low immunogenicity, and the capacity to deliver large fragments of DNA.

However, several studies (for example, Mahato et al., J. Pharm. Sci., 1995, 84, 1267-1271, Thierry et al., 1995, PNAS 92, 9742-9746) have shown that the transfer efficiency of the complexed anionic macromolecules into the cells, especially in the case of *in vivo* transfer, can vary in function of the interaction between the complexes and the cell membranes, the cell type involved, the lipid composition of the cationic components, the size of the complexes formed with the anionic molecules and, especially, the ratio of the positive to negative charges of the different components of the complex. Currently, very little is known concerning the mechanisms which enable the interaction of the complexes with the cell membranes and the transfer of the complexes into the cell, and the ongoing research remains highly empirical. In addition, such compounds are non-self molecules and may then be recognized by the immune system and/or interfere with metabolic pathways. Consequently, there is a need to develop new compounds, especially cationic compounds, with improved properties or properties different from those already described or which may be combined with the previous art.

The applicant has now identified new complexes which are capable of transfering at least one anionic substance of interest, in particular a nucleic acid, into a cell and which can find an *in vivo* gene therapy application.

The present invention first concerns a complex for transfering an anionic substance of interest into a cell comprising :
i) at least a first polypeptide capable of binding to an anionic substance,
ii) an anionic substance of interest,
wherein said first polypeptide comprises all or part of an amino acid sequence selected from the group consisting of the subunit amino acid sequences of the C1 complement factor.

The immune system of complement is an extremely complex combination of serum proteins or factors present at low concentration in normal serum of all vertebrates. Said complement factors are naturally present in an inactive form and can be activated to form an elaborated enzymatic pathway (classical C pathway) including at least nine factors designated by C1 to C9 (see Weir and Stewart, 1993, Immunology, pages 23-30, 7^{th} Edition, Churchill Livingstone).

C1 is one of said complement factors. It is a polypeptide comprising at least three subunits (C1q, C1r and C1s) associated in the presence of ionic calcium. C1q has been widely analyzed : it is composed of 18 chains (three different chains termed A, B and C with six copies of each per C1q molecule) and shows a molecular weight of about 400000.

Although DNA has been previously shown to activate the classical C pathway (Peltier et al., 1978, Immunology, 35, 779) and Jiang et al. (1992, J. of Exp. Medicine, 175, 1373-1379) have for the first time delineated the C1q regions through which said classical C pathway can be activated, suggesting that DNA can interact with said C1q regions, use of such compound has neither been taught nor suggested for transferring an anionic substance of interest into a cell.

"Anionic substance of interest" designates a negatively-charged molecule without number of charges limitation. More specifically said molecule can be selected from the group consisting of proteins or nucleic acids. According to a preferred embodiment, said anionic substance of interest is a nucleic acid.

The term "nucleic acid" as used in the scope of the present invention means a DNA and/or RNA or fragments thereof, single or double-stranded, linear or circular, natural or synthetic, modified or not (see US 5525711, US 4711955, US 5792608 or EP 302 175 for modification examples) without size limitation. It may, *inter alia,* be a genomic DNA, a cDNA, an mRNA, an antisense RNA, a ribozyme, or a DNA encoding such RNAs. "Polynucleotides" and "nucleic acids" are synonyms with regard to the present invention. The nucleic acid may be in the form of a linear polynucleotide, and preferably in the form of a plasmid. The nucleic acid can also be an oligonucleotide which is to be delivered to the cell, e.g., for antisense or ribozyme functions. According to the invention, the nucleic acid is preferably a naked polynucleotide (Wolff et al., Science 247 (1990), 1465-1468) or is formulated with at least one compound such as a polypeptides, preferably viral polypeptides, or cationic lipids, or cationic polymers which can participate in the uptake of the polypeptide into the cells (see Ledley, Human Gene Therapy 6 (1995), 1129-1144 for a review) or a protic polar compound (examples are provided below in the present specification or in EP 890362). Preferably, said nucleic acid includes at least one therapeutically useful gene sequence that can be transcribed and translated to generate a polypeptide of interest and the elements enabling its expression. The genetic information necessary for expression by a target cell comprises all the elements required for transcription of DNA into RNA and, if necessary, for translation of mRNA into a polypeptide. Transcriptional promoters suitable for use in various vertebrate systems are well known. For example, suitable promoters include viral promoters like RSV, MPSV, SV40, CMV or 7.5k, vaccinia promoter, inducible promoters, tissue specific promoters, synthetic promoters, etc or combinations thereof. The nucleic acid can also include intron sequences, targeting sequences, transport sequences, sequences involved in replication or integration. Said sequences have been reported in the literature and can readily be obtained by those skilled in the art. The nucleic acid can also be modified in order to be stabilized with specific components as spermine. It can also be substituted, for example by chemical modification, in order to facilitate its binding with specific polypeptides such as, for example the first and/or second polypeptides of the present invention. Such substitutions are exemplified below. According to the invention, the nucleic acid can be homologous or heterologous to the target cells into which it is introduced. Examples of polypeptides encoded by the nucleic acid are enzymes, hormones, cytokines, membrane receptors, structural polypeptides, transport polypeptides, albumin, tumoral, viral or infectious antigens, adhesines, ligands, transcription factors, translation factors, replication factors, stabilization factors, antibodies, E6 or E7 from HPV, MUC1, BRCA1, interferons, interleukin (IL-2, IL-4, IL-6, IL-7, IL-12, GM-CSF (Granulocyte Macrophage Colony Stimulating Factor), the tk gene from Herpes Simplex type 1 virus (HSV-1), p53 or VEGF. The polynucleotide can also code for an antibody. In this regard, the term "antibody" encompasses whole immunoglobulins of any class, chimeric antibodies and hybrid antibodies with dual or multiple antigen or epitope specificities, and fragments, such as F(ab)'₂, Fab', Fab including hybrid fragments and anti-idiotypes (US 4,699,880). Advantageously said nucleic acid encodes all or part of a polypeptide which is an immunity conferring polypeptide and acts as endogenous immunogen to provoke a humoral or cellular response, or both, against infectious agents, including intracellular viruses, and also against tumor cells. An "immunity-conferring polypeptide" means that said polypeptide when it is produced in the transfected cells will participate in an immune response in the treated patient. More specifically, said polypeptide produced in or taken up by macropinocyte cells such as APCs will be processed and the resulting fragments will be presented on the surface of these cells by MHC class I and/or II molecules in order to elicit a specific immune response.

The introduction or transfer process of an anionic substance of interest into a cell is by itself well known. "Introduction or transfer" means that the anionic substance is transferred into the cell and is located, at the end of the process, inside said cell or within or on its membrane. If the anionic substance is a nucleic acid, this is called "transfection". Transfection can be verified by any appropriate method, for example by measuring the expression of a gene encoded by said nucleic acid or by measuring the concentration of the expressed protein or its mRNA, or by measuring its biological effect.

"Capable of binding to" means that the considered compound is capable to interact and to bind to another compound, preferably in a reversible manner, for example by ionic interactions, by forming disulfide or hydrogene bonds, by hydrophobic interactions or covalent bonds. According to a particular embodiment, a polypeptide of the invention is capable to interact and to bind to an anionic substance of interest, and preferably to a single stranded and/or double stranded nucleic acid. Preferably, a polypeptide of the invention is capable to interact and to bind to an anionic substance of interest at least by the intermediate of ionic interactions. Moreover, said interaction/binding can occur naturally or after modification or substitution of said polypeptide or of said anionic substance of interest, directly or bound to each other by a linker group. Examples of such modifications or substitutions are provided below. Moreover, said binding can be targeted, specifically or unspecifically. In a particular embodiment, "capable of binding to" means that the interaction/binding occurs by the formation of a disulfide bond. In this specific case, polypeptides are involved in said binding, for example said first and second polypeptides. The disulfide bond can be formed by a reaction between cysteine residues carried by said first and/or second polypeptide, or substituted on said anionic substance of interest. Said disulfide bonds are labile *in vivo* and can be naturally cleaved after transfer of the anionic substance into a cell. This embodiment is of particular interest for limiting a possible immune response directed against macromolecules of the complex consisting of various polypeptides which by themselves are not immunogenic but were associated to other molecules.

According to a particular embodiment, the polypeptide of the complex comprises all or part of an amino acid sequence selected from the group consisting of the C1q, C1r and C1s amino acid sequence subunits, and advantageously from the group consisting of the C1qA, C1qB and C1qC amino acid sequences of the C1q subunit .

In a more preferred embodiment, the polypeptide of the complex comprises all or part of an amino acid sequence selected from the group consisting of the amino acid sequences set forth in SEQ ID NO : 1, SEQ ID NO : 2 and SEQ ID NO :3 .

According to the present invention, the complex may further comprise :
(i) at least a second polypeptide involved in cell-specific targeting and/or in membrane destabilisation and/or which promotes said transfer of an anionic substance of interest into a cell and/or
(ii) at least one cationic compound selected from the group consisting of cationic lipids and cationic polymers and/or
(iii) at least one negatively charged, neutral or zwitterionic lipid selected from phosphatidylethanolamine (PE), phosphatidylcholine, phosphocholine, sphingomyelin, ceramide, cerebroside, dioleylphosphatidylethanolamine (DOPE) and one of their derivatives.

Said second polypeptide may be present in the complex of the invention as an independent polypeptide (not bound to said first polypeptide or said anionic substance) or as a polypeptide capable of binding to said first polypeptide or said anionic substance of interest. In cases where it is capable of binding, said second polypeptide is naturally able to bind to its target (ligand/antiligand model)or is modified in order to facilitate or allow said binding to occur. Modification of the molecules are exemplified below. The second polypeptide preferably is capable of binding to the first polypeptide mediated by at least one disulfide bond.

The polypeptidic and anionic parts of the present complex may be modified or substituted by chemical or natural processes widely used by the skilled man in order to obtain compounds modified or substituted with : labeling molecules (for example, see molecules disclosed in US-A-4,711,955) enabling, for example, visualization of the distribution of the polypeptide, or of the nucleic acid, or of the complex of the invention, after *in vitro* or *in vivo* administration; cell targeting molecules (ligands) or anchoring molecules ; elements facilitating penetration into the cell, lysis of endosomes or even intracellular transport towards the nucleus. These elements may be composed of all or part of sugars, glycol, peptides (e.g. GRP, Gastrin Releasing Peptide, JTS1 described in Gottschalk et al, 1996, Gene Therapy, 3, 448-457 ; SEQ ID NO:4), oligonucleotides, lipids (especially those with C2-C22), hormones, vitamins, antigens, antibodies (or fragments thereof), specific membrane receptor ligands, ligands capable of reaction with an anti-ligand, fusogenic peptides, nuclear localization peptides, or a combination of said compounds, e.g. galactosyl residues to target the asialoglycoprotein receptor on the surface of hepatocytes, the INF-7 fusogenic peptide derivated from the HA-2 subunit of the influenza virus hemagglutinin (Plank et al. 1994, J. Biol. Chem. *269*, 12918-12924) for membrane fusion, or pore forming peptides such as staphylococcal alpha and delta-toxins, magainins, cecropins, streptolysine O, cnidarian toxins, perforin, and aerolysin (Ojcius at al, 1991, TIBS 16, 225-228) or a nuclear signal sequence derived from the T-antigen of the SV40 virus (Lanford and Butel, 1984, Cell 37, 801-813) or from the EBNA-1 protein of the Epstein Barr virus (Ambinder et al., 1991, J. Virol. 65, 1466-1478).

According to a specific embodiment, said second polypeptide comprises all or part of the sustituting or modifying polypeptides cited above. In a preferred embodiment, said second polypeptide comprises all or part of an amino acid sequence of an anti-DNA antibody (such as P1 and P2 peptides described in Avrameas et al, 1998, PNAS, 95, 601-606), of the JTS1 peptide described in Gottschalk et al. (1996, Gene Therapy, 3, 448-457) or of a polylysine polypeptide. Advantageously, said second polypeptide comprises all or part of an amino acid sequence set forth in SEQ ID NO:4, SEQ ID NO:5 or SEQ ID NO:6.

The complex of the invention may further comprise (ii) at least one cationic compound selected from the group consisting of cationic lipids and cationic polymers. These cationic compounds are widely described in the scientific literature (see for example the references related to non-viral delivery systems mentioned above, or patent applications n° WO 97/29118, WO 98/08489, WO 98/17693. Cationic lipids or mixtures of cationic lipids which may be used in the present invention include cationic lipids selected from the group consisting of Lipofectin™, DOTMA: N-[1-(2,3-dioleyloxyl)propyl]-N,N,N-trimethylammonium (Felgner, PNAS 84 (1987), 7413-7417), DOGS: dioctadecylamidoglycylspermine or Transfectam™ (Behr, PNAS 86 (1989), 6982-6986), DMRIE: 1,2-dimiristyloxypropyl-3-dimethyl-hydroxyethylammonium and DORIE: 1,2-diooleyloxypropyl-3-dimethylhydroxyethylammnoium(Felgner, Methods 5 (1993), 67-75), DC-CHOL: 3 [N-(N',N'-dimethylaminoethane)-carbamoyl]cholesterol (Gao, BBRC 179 (1991), 280-285), DOTAP (McLachlan, Gene Therapy 2 (1995), 674-622), Lipofectamine™, spermine or spermidine-cholesterol, Lipofectace™ (for a review see for example Legendre, Medecine/Science 12 (1996), 1334-1341 or Gao, Gene Therapy 2 (1995), 710-722), cationic lipid as disclosed in patent applications WO 98/34910, WO 98/14439, WO 97/19675, WO 97/37966, FR 97 02420 (filing number)or FR 97 07290 (filing number) and their isomers. Nevertheless, this list is not exhaustive and other cationic lipids well known in the art could be used in the method of the invention as well.

Preferably, the cationic lipids of the present invention are selected from among cationic lipids of the formula (WO 98/34910): 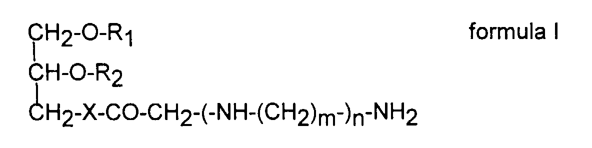 wherein :
R₁, R₂, are identical or different and are C₆-C₂₃ alkyl or alkenyl, linear or branched, or -C(=O)-(C₆-C₂₃)alkyl or -C(=O)-(C₆-C₂₃)alkenyl, linear or branched,
X is O or -NR₃, R₃ being H or C₁-C₄ alkyl,
n = 1 to 6,
m = 1 to 6, and when n > 1, m can be identical or different.

According to another embodiment, the cationic lipid is selected from cationic lipids of the formula (FR 97 02420 filing number):

R-HN-[-(CH₂)ₘ-NR-]ₙ₋₁-(CH₂)ₘ-NH-R formula II

wherein:
R is, independently of one another, H or 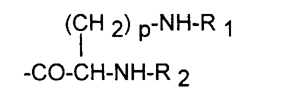 wherein :
R₁ and R₂ are, independently of one another C₆-C₂₃ alkyl or alkenyl, linear or branched, or -C(=O)-(C₆-C₂₃) alkyl or -C(=O)-(C₆-C₂₃)alkenyl, linear or branched, aryl, cycloalkyl, fluoroalkyl, polyethylene glycol, oxyethylene ou oxymethylene radicals,
p= 1 to 4,
n = 1 to 6,
m =1 to 6.

Cationic polymers or mixtures of cationic polymers which may be used in the present invention include cationic polymers selected from the group consisting of chitosan, poly(aminoacids) such as polylysine (US-A-5,595,897 and FR 2 719 316); polyquaternary compounds; protamine; polyimines;polyethylene imine or polypropylene imine (WO 96/02655) ; polyvinylamines; polycationic polymer derivatized with DEAE, such as pullulans, celluloses; polyvinylpyridine; polymethacrylates; polyacrylates; polyoxethanes; polythiodiethylaminomethylethylene (P(TDAE)); polyhistidine; polyornithine; poly-p-aminostyrene; polyoxethanes; co-polymethacrylates (eg copolymer of HPMA; N-(2-hydroxypropyl)-methacrylamide); compound disclosed in US-A-3,910,862, polyvinylpyrrolid complexes of DEAE with methacrylate, dextran, acrylamide, polyimines, albumin, onedimethylaminomethylmethacrylates and polyvinylpyrrolidonemethylacrylaminopropyltrimethyl ammonium chlorides; polyamidoamines; telomeric compounds (patent application filing number EP 98.401471.2). Nevertheless, this list is not exhaustive and other cationic polymers well known in the art can be used in the method of the invention as well.

According to a particular embodiment, the cationic polymer is a substituted polyvinylamine (PCT patent application, filing number PCT/FR98/01581) such as defined by formula: 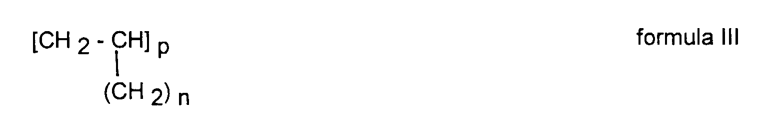 wherein n =0 to 5, p = 2 to 20000
wherein:
- at least 10% of free NH₂ are substituted with R, and R is an hydrophilic group.

According to another embodiment said cationic polymer is a polymer of the general formula (European patent application, filing number 98/402142.8): 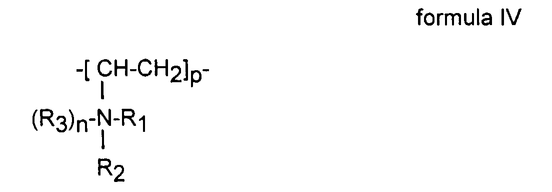 wherein:
the degree of polymerization p ranges from 2 to 1000000;
R₁, R₂ and R₃, independently of one another in each [CH - CH₂] repeat, are selected from H, alkyl of 1 to 20 carbon atoms or aryl of 5 to 7 carbon atoms; n is 0 or 1, with the proviso that at least one n is 1 in the full length of the polymer.

Colipids are optionally included in the complexes of the invention in order to facilitate entry of the nucleic acid into the cell. According to the invention, colipids are selected from the group consisting of positively or negatively charged, neutral and zwitterionic lipids. These colipids could be natural or synthetic compounds, or a combination of each.

Preferably, the colipid is selected from the group consisting of phosphatidylethanolamine (PE), phosphatidylcholine, phosphocholine, dioleylphosphatidylethanolamine (DOPE), sphingomyelin, ceramide or cerebroside or one of their derivatives.

The ratios of cationic transfecting molecules to colipid (on a weight to weight basis), when the two compounds are co-existing in the complex, can range from 1:0 to 1:10. In preferred embodiments, the ratio ranges from 1:0.5 to 1:4.

In a specific embodiment of the invention, the size of the complex according to the invention is small (less than 2µm, more advantageously less than 500 nm and, preferably, it ranges between 20 and 100 nm). A complex size may be selected for optimal use in particular applications. Measurements of the complex size can be achieved by a number of techniques including, but not limited to, dynamic laser light scattering (photon correlation spectroscopy, PCS), as well as other techniques known to those skilled in the art (see, Washington, Particle Size Analysis in Pharmaceutics and other Industries, Ellis Horwood, New York, 1992, 135-169). Sizing procedure may also be applied on complexes in order to select specific complex sizes. Methods which can be used in this sizing step include, but are not limited to, extrusion, sonication and microfluidization, size exclusion chromatography, field flow fractionation, electrophoresis and ultracentrifugation.The complexes of the invention may also be characterized by their theoretical charge ratio (+/-), which is the ratio of the positive charges provided by at least the first polypeptide to the negative charges provided by the anionic substance in the complex, assuming that all potentially cationic groups are in fact in the cationic state and all potentially anionic groups are in fact in the anionic state. In general, an excess of positive charges on the complex facilitates binding of the complex to the negatively-charged cell surface. To obtain such a ratio, the calculation shall take into account all negative charges in the anionic substance and shall then adjust the quantity of polypeptide necessary to obtain the desired theoretical charge ratio indicated above. The quantities and the concentrations of the other ingredients shall be adjusted in function of their respective molar masses and their number of positive charges. The ratio is not specifically limited: quantities are selected so that the ratio between the number of positive charges in the polypeptide and the number of negative charges in the anionic substance is between 0.05 and 20, preferably between 2.5 and 15, and most preferably around 2.5 to 10. Furthermore, the concentration of the negatively-charged anionic substance, which may be added to the polymer to form said complexes of the invention may range from 10 µg/ml to 5000 µg/ml. In a preferred embodiment of the invention, the concentration of anionic substance ranges from 100 µg/ml to 1000 µg/ml

The invention is also directed to a process for the preparation of the above described complexes, comprising the following steps:
- contacting at least a first polypeptide capable of binding to an anionic substance and comprising all or part of an amino acid sequence selected from the group consisting of the subunit amino acid sequences of the C1 complement factor, with at least one anionic substance of interest,
- and recovering said complex, optionally after a purification step.

Where the complex of the invention further comprises :
(i) at least a second polypeptide involved in cell-specific targeting and/or in membrane destabilisation and/or which promotes said transfer of an anionic substance of interest into a cell and/or
(ii) at least one cationic compound selected from the group consisting of cationic lipids and cationic polymers and/or
(iii) at least one negatively charged, neutral or zwitterionic lipid ,
said process comprises the steps of:
- first mixing said first polypeptide with said additional element (i) and/or (ii) and/or (iii) and then complexing the anionic substance of interest, or
- first complexing said first polypeptide with the anionic substance of interest and then mixing the complex with said additional element (i) and/or (ii) and/or (iii).

The process can further comprise a sizing procedure as described above.

The invention also encompasses a pharmaceutical composition for transfering an anionic substance of interest into a cell, wherein said composition comprises at least one complex as previously disclosed. Said pharmaceutical compositions are particularly useful for the delivery of polynucleotides to cells or tissues of a subject in gene therapy methods but are not limited to such use. The term "gene therapy method" is preferably understood as a method for the introduction of a polynucleotide into cells either *in vivo* or by introduction into cells *in vitro* followed by re-implantation into a subject. "Gene therapy" in particular concerns the case where the gene product is expressed in a target tissue as well as the case where the gene product is excreted, especially into the blood stream.

Preferably, the pharmaceutical composition furthermore comprises a pharmaceutically acceptable carrier. The carrier is preferably isotonic, hypotonic or weakly hypertonic and has a relatively low ionic strength, such as provided by a sucrose, glucose, solution or artificial serum. Furthermore, it may contain any relevant solvents, aqueous or partly aqueous liquid carriers comprising sterile, pyrogen-free water, dispersion media, coatings, and equivalents. The pH of the pharmaceutical preparation is suitably adjusted and buffered.

The invention more particularly relates to a pharmaceutical composition comprising at least one of the complexes described above and also incorporating at least one adjuvant capable of improving the transfection capacity of said complex. Adjuvants may be selected from the group consisting of a chloroquine, protic polar compounds, such as propylene glycol, polyethylene glycol, glycerol, EtOH, 1-methyl L -2-pyrrolidone or their derivatives, or aprotic polar compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile and their derivatives.

The pharmaceutical composition of the present invention can be administered into a vertebrate tissue. This administration may be made by intradermal, subdermal, intravenous, intramuscular, intranasal, intracerebral, intratracheal, intraarterial, intraperitoneal, intravesical, intrapleural, intracoronary or intratumoral injection, by means of a syringe or other devices. Transdermal administration is also contemplated, as are inhalation, aerosol routes, instillation or topical application.

According to the present invention, the pharmaceutical composition can be administered into target tissues of the vertebrate body including those of muscle, skin, brain, lung, liver, spleen, bone marrow, thymus, heart, lymph, bone, cartilage, pancreas, kidney, gall bladder, stomach, intestine, testis, ovary, uterus, rectum, nervous system, eye, gland, connective tissue, blood, tumor, etc.

Applied to *in vivo* gene therapy, this invention allows repeated administration to the patient without any risk of the administered preparation to induce a significant immune reaction. Administration may be by single or repeated dose, once or several times after a certain period of time. Repeated administration allows a reduction of the dose of active substance, in particular DNA, administered at a single time. The route of administration and the appropriate dose varies depending on several parameters, for example the individual patient, the disease being treated, or the nucleic acid being transferred.

According to the invention, "cells" include prokaryotic cells and eukaryotic cells, yeast cells, plant cells, human or animal cells, in particular mammalian cells. In particular, cancer cells should be mentioned. The invention can be applied *in vivo* to the interstitial or luminal space of tissues in the lungs, the trachea, the skin, the muscles, the brain, the liver, the heart, the spleen, the bone marrow, the thymus, the bladder, the lymphatic system, the blood, the pancreas, the stomach, the kidneys, the ovaries, the testicles, the rectum, the peripheral or central nervous system, the eyes, the lymphoid organs, the cartilage, or the endothelium. In preferred embodiments, the cell will be a muscle cell, a hematopoietic system stem cell or an airways cell, more especially a tracheal or pulmonary cell, and preferably a cell of the respiratory epithelium.

The present invention also encompasses a process for transfering a nucleic acid into cells wherein said process comprises contacting said cells with at least one complex or composition according to the invention. This process may be applied by direct administration of said complex or composition to cells of the animal *in vivo,* or by *in vitro* treatment of cells which were recovered from the animal and then re-introduced into the animal body (*ex vivo* process). In *in vitro* application, cells cultivated on an appropriate medium are placed in contact with a suspension consisting of complexes or composition of the invention. After an incubation time, the cells are washed and recovered. Introduction of the active substance can be verified (eventually after lysis of the cells) by any appropriate method.

In the case of *in vivo* treatment according to the invention, in order to improve the transfection rate, the patient may undergo a macrophage depletion treatment prior to administration of the pharmaceutical preparations described above. Such a technique is described in the literature (refer particularly to Van Rooijen et al., 1997, TibTech, 15, 178-184).

The invention further concerns the use of a complex as defined above for the preparation of a pharmaceutical composition for curative, preventive or vaccinal treatment of man or animals, and more specifically for gene therapy use.

The invention also concerns the use of all or part of at least one amino acid sequence corresponding to the subunits of the C1 complement factor, especially C1q, C1r and C1s, for the preparation of a complex for transferring an anionic substance of interest into a cell. According to a preferred embodiment, said amino sequence is selected from the group consisting of the C1qA, C1qB and C1qC amino acid sequences of C1q subunit.

These and other embodiments are disclosed or are obvious from and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on Internet, e.g. under http://www.ncbi.nlm.nih.gov/PubMed/medline.html. Further databases and addresses, such as http://www.ncbi.nlm.nih.gov, http://www.infobiogen.fr, http://www.fmi.ch/biology/research_tools.html, http://www.tigr.org. are known to the person skilled in the art and can also be obtained using, e.g., http://www.lycos.com. An overview of patent information in biotechnology and a survey of relevant sources of patent information useful for retrospective searching and for current awareness is given in Berks, TIBTECH 12 (1994), 352-364.

The methods, compositions and uses of the invention can be applied in the treatment of all kinds of diseases the treatment and/or diagnostic of which is related to or dependent on the transfer of nucleic acids in cells. The compositions, and uses of the present invention may be desirably employed in humans, although animal treatment is also encompassed by the uses described herein.

The invention has been described in an illustrative manner, and it is to be understood that the terminology which has been used is intended to be in the nature of words of description rather than of limitation. Obviously, many modifications and variations of the present invention are possible in light of the above teachings. It is therefore to be understood that within the scope of the appended claims, the invention may be practiced different from what is specifically described herein. The mentioned articles or literatures are interpreted hereby by reference in their entity.

Figure 1 : Levels at which the gene encoding luciferase is expressed (RLU/mg of protein) : comparison of the transfection efficiency of complexes comprising C1qA or K8 and plasmid pTG11033 in 293 EBNA1 cells.

Figure 2 : Levels at which the gene encoding luciferase is expressed (RLU/mg of protein) : transfection efficiency of complex comprising plasmid pTG11033 and C1qA-P3 in 293EBNA cells.

### EXAMPLES

### Cells

293 EBNA cells (Invitrogen) are maintenained in culture in DMEM medium supplemented with 10% fetal calf serum, gentamycine 1%, glutamine 1%, G418 250 µg/ml and 3 g/l glucose, in an incubator at 37 C and 5% CO ₂. 24 hours prior to transfection cells are seeded in 6 multi-wells plate at a concentration of 2,5.10⁵ cells/well.

### Plasmid

Plasmid pTG110S6 (Längle-Rouaulrotal, J. of Virology 72 (1998), 6181-6185)is used which includes the gene encoding luciferase placed under the control of the CMV promoter, intron 1 of the HMG gene and a SV40 polyA signal.

### Polypeptides

The polypeptides were purchased from ALTERGEN in milliQ water.

### Example 1: Preparation of Polypeptide /DNA complexes and protocol of transfection

The Polypeptide/DNA complexes are prepared as follows : 3 µg of plasmid DNA and the desired amount of tested polypeptide charge ratio = 1:4 ,1 N/P equivalent corresponds to the amount of peptide necessary to have one amino group (N) per phosphate group (P) of nucleic acid (330 Da mean MM)] are mixed in 750 µl 10% sucrose or 5% glucose or deproteinated serum solution and vortexed. After incubation for 30 min at room temperature, the complexes are added to the cells.

Before adding the transfection complexes, the medium (DMEM, GIBCO BRL) is removed and replaced by 2 ml of serum-free medium and 750 µl of the complex solution.

After 24 h, the medium is removed, 250 µl of lysis buffer (Promega) are added and cells are frozen at -80° C until measurement of luciferase activity.

20 I of the supernatant are analyzed using the luciferase assay system (Promega) in 96 multi-well plates (Biolumat LB 9500, Berthold, Wilbach, Germany). Each transfection is done in triplicate. Values are given as mean relative light units (RLU) per mg of cell protein (BCA assay, Pierce). The luciferase activity which is measured is standardized in relation to the quantity of protein with the aid of a standard scale which is generated using commercially available luciferase (Promega). The quantity of total protein is also determined by means of the bicinchoninic acid (BCA) colorimetric method (Smith, Anal. Biochem. 150 (1985), 76-85) using an aliquot of supernatant. This allows to express the luciferase activity in RLU per milligram of protein extracted from the cells.

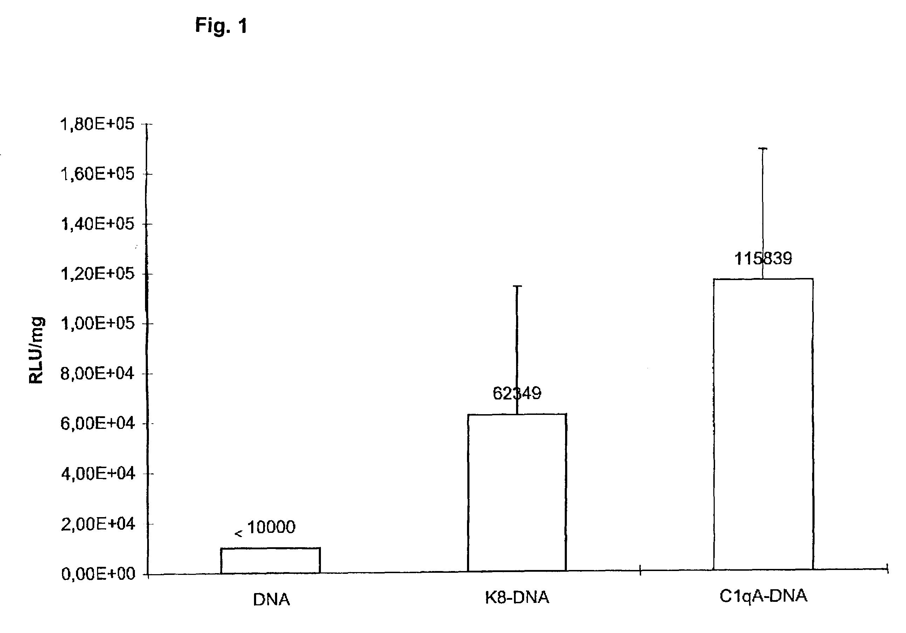

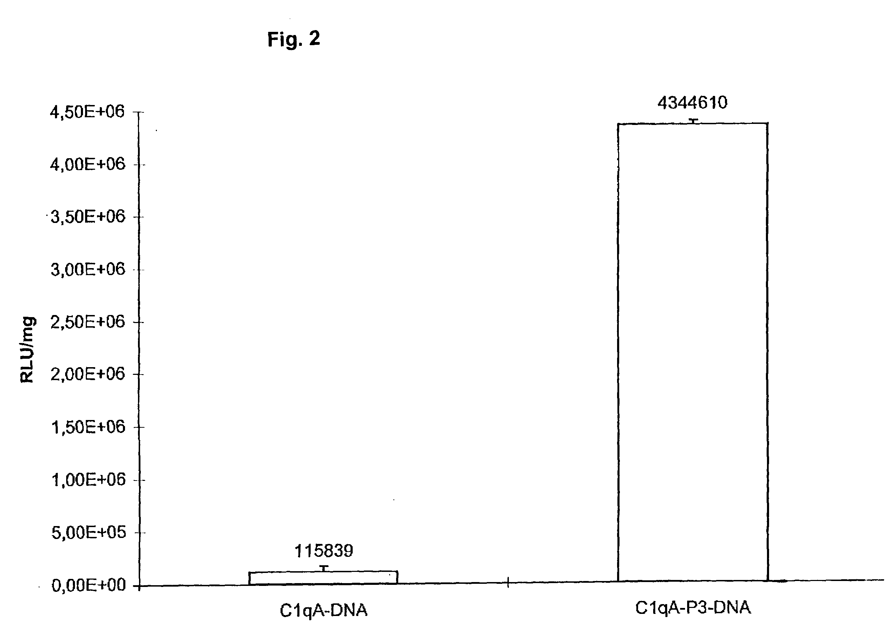

Results are presented in Figures 1 and 2.

Figure 1 presents the transfection efficiency of a complex comprising the polypeptide K8 compared to a complex comprising the polypeptide C1qA. K8 is a synthetic polylysine chain comprising 10 lysine residues (SEQ ID NO:5). C1qA is a synthetic polypeptide consisting of SEQ ID NO:1. The K8-DNA and C1qA-DNA complexes are formed according to the method presented above. DNA alone is used as a control. This experiment clearly shows that the C1qA-DNA complex is at least as efficient as the K8-DNA complex for transfering said DNA into the 293EBNA1 cells.

Figure 2 presents the transfection efficiency of a complex comprising a first polypeptide C1qA and a second polypeptide P3. The polypeptide C1qA-P3 is obtained from Altergen (Schiltigheim - France). The N-terminal end of C1qA is associated to the C-terminal end of C1qA by the mean of a peptide bond. The DNA-C1qA-P3 complex is formed as previously described and its transfering property is compared to those of a complex comprising only P3. The observed results clearly indicate that the combination of said first (C1qA) and second (P3) polypeptides in the complex with DNA strongly improves transfection properties of said complex.

Moreover, various charge ratios have been tested which indicates that optimal transfection is obtained with positive/negative charge ratio between 4 and 10.

### Example 2: Interaction between peptide C1qA(4-26) and plasmid pTG11033 has been shown after electrophoresis on agarose gel

Gel retardation assay was performed with C1qA (4-26) in several buffers: PBS, TBS, 5% glucose, or ultrafiltrated serum (YM10 Amicon membrane). DNA and peptides have been mixed at charge ratios [-/+] of [1:4], [1:1] and [1:0.2]. It appeared that the [1:4] charge ratio led in every case to a strong association of DNA to the peptides, characterized by an absence of visible band in the presence of ethidium bromide, after gel-electrophoresis (0.8% agarose, TBE). For the [1:1] charge ratio complex, we could show a significative retardation in DNA uncomplexed plasmid.

### Example 3: Sizes of complexes have been evaluated by light-scattering.

Sizes of complexes prepared with a plasmid DNA (PTG11033; see EP-A 890 32, 9.6 kb) and the C1qA (4-26) peptide (see Table I) were evaluated. Complexes were formulated either in 5% glucose or in ultrafiltrated foetal calf serum (YM10 Amicon membrane). Formulation was made in 500 µl buffer, with 6 µg DNA and the quantity of peptide necessary to form a [1:4], [-:+] complex. As a control a polylysine SV40 NLS peptide was used. (Seq: H2N-bio-Tyr-Lys-Ala-Lys-Lys-Lys-Lys-Lys-Lys-Lys-Lys-Trp-Lys-Gly-Gly-Pro-Lys-Lys-Lys-Arg-Lys-Val-COOH) The size measurement (see Table I) showed an advantage for the use of C1qA in ultrafiltrated serum compared to 5% glucose. When oxidized, the dimerized C1qA peptide could form even smaller complexes, whatever the formulation buffer used. The reasonable sizes obtained in ultrafiltrated serum with C1qA peptides is an advantage for the use of this peptide rather than polylysine derivatives.

**Table I**

| Peptide | Sizes in 5% glucose | Sizes in ultrafiltrated serum |
|---|---|---|
| SP-1052 (C1qA, 4-26) | 1µm | 300-500 nm |
| SP-1052 oxidized | 200 nm | 300 nm |
| bioY22V | 100 nm | 1-2 µm |

Complexes (-/+):[1:4], 6 µg DNA, 500 µl.

Size measures: quasi-elastic light scattering, COULTER N4+.

## Claims

1. A complex for transfering an anionic substance of interest into a cell comprising:
(i) at least a first polypeptide capable of binding to an anionic substance,
(ii) an anionic substance of interest,
wherein said first polypeptide comprises all or part of an amino acid sequence selected from the group consisting of the subunit amino acid sequences of the C1 complement factor.

2. The complex of claim 1, wherein said amino acid sequence is selected from the group consisting of the C1q, C1r and C1s amino acid sequence subunits.

3. The complex of claim 2 wherein said amino acid sequence is selected from the group consisting of the C1qA, C1qB and C1qC amino acid sequences of the C1q subunit.

4. The complex of claim 3, wherein said amino acid sequence is selected from the group consisting of amino acid sequences set forth in SEQ ID NO :1, SEQ ID NO : 2 or SEQ ID NO :3.

5. The complex of any one of claims 1 to 4, wherein said complex further comprises :
(i) at least a second polypeptide involved in cell-specific targeting and/or in membrane destabilisation and/or which promotes said transfer of an anionic substance of interest into a cell and/or
(ii) at least one cationic compound selected from the group consisting of cationic lipids and cationic polymers and/or
(iii) at least one negatively charged, neutral or zwitterionic lipid selected from phosphatidylethanolamine (PE), phosphatidylcholine, phosphocholine, sphingomyelin, ceramide, cerebroside, dioleylphosphatidylethanolamine (DOPE) and one of their derivatives.

6. The complex of claim 5, wherein said second polypeptide comprises all or part of an amino acid sequence set forth in SEQ ID NO:4, SEQ ID NO:5 or SEQ ID NO:6.

7. The complex of any one of claims 1 to 6, wherein said anionic substance of interest is a nucleic acid or a protein.

8. The complex of claim 7, wherein said anionic substance of interest is a nucleic acid selected from the group consisting of cDNA, genomic DNA, plasmid DNA, messenger RNA, antisense RNA, ribozymes, transfer RNA, ribosomal RNA and DNA encoding for such types of RNA.

9. The complex of claim 8, wherein said nucleic acid includes at least one therapeutically useful gene sequence and the elements enabling its expression.

10. The complex of any one of claims 1 to 9, wherein said complex size is less than 500 nm.

11. The complex of claim 10, wherein said complex size is between 20 and 100 nm.

12. The complex of any one of claims 1 to 11, wherein the ratio between the number of positive charges and the number of negative charges is between 0,05 and 20, preferably between 2,5 and 15, and most preferably about 4 to 10,0.

13. A process for the preparation of a complex of any one of claims 1 to 12, comprising the following steps:
- contacting at least a first polypeptide capable of binding to an anionic substance and comprising all or part of an amino acid sequence selected from the group consisting of the subunit amino acid sequences of the C1 complement factor, with at least one anionic substance of interest,
- and recovering said complex, optionally after a purification step.

14. A process for the preparation of a complex of any one of claims 5 to 12, comprising the following steps:
- first mixing said first polypeptide with said additional element (i) and/or (ii) and/or (iii) and then complexing the anionic substance of interest, or
- first complexing said first polypeptide with the anionic substance of interest and then mixing the complex with said additional element (i) and/or (ii) and/or (iii).

15. A pharmaceutical composition for transferring an anionic substance of interest into a cell comprising at least one complex of any one of claims 1 to 12.

16. The pharmaceutical composition of claim 15, further comprising at least one adjuvant.

17. The pharmaceutical composition of claim 16, wherein said adjuvant is selected from the group consisting of chloroquine, protic polar compounds such as propylene glycol, polyethylene glycol, glycerol, ethanol, 1-methyl L -2-pyrrolidone or their derivatives, or aprotic polar compounds such as dimethylsulfoxide (DMSO), diethylsulfoxide, di-n-propylsulfoxide, dimethylsulfone, sulfolane, dimethylformamide, dimethylacetamide, tetramethylurea, acetonitrile and their derivatives.

18. Use of a complex of any one of claims 1 to 12 for the preparation of a pharmaceutical composition for curative, preventive or vaccinal treatment of man or animals, preferably for gene therapy.

19. The use of claim 18, wherein said pharmaceutical composition is administered by intramuscular injection, by inhalation, by intratracheal injection, by instillation, by aerosolization or by topical application.

20. Use of all or part of at least one amino acid sequence corresponding to the subunits of the C1 complement factor for the preparation of a complex for transfering an anionic substance of interest into a cell.


SEQUENCE LISTING

(1) GENERAL INFORMATION:

(i) APPLICANT:
(A) NAME: TRANSGENE SA
(B) STREET: 11 rue de Molsheim
(C) CITY: Strasbourg
(E) COUNTRY: FRANCE
(F) POSTAL CODE (ZIP): 67000
(G) TELEPHONE: 03 88 27 91 00
(H) TELEFAX: 03 88 27 91 41

(ii) TITLE OF INVENTION: Antitumoral complexes

(iii) NUMBER OF SEQUENCES: 6

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(2) INFORMATION FOR SEQ ID NO:1:

(i) SEQUENCE CHARACTERISTICS:
(A) LENGTH: 23 amino acids
(B) TYPE: amino acid
(D) TOPOLOGY: unknown

(ii) MOLECULE TYPE: peptide

(v) FRAGMENT TYPE: internal

(vi) ORIGINAL SOURCE:
(A) ORGANISM: Homo sapiens

(xi) SEQUENCE DESCRIPTION: SEQ ID NO: 1:

Cys Arg Ala Pro Asp Gly Lys Lys Gly Glu Ala Gly Arg Pro Gly Arg
1               5                   10                  15

Arg Gly Arg Pro Gly Leu Lys
            20

(2) INFORMATION FOR SEQ ID NO: 2:

(i) SEQUENCE CHARACTERISTICS:
(A) LENGTH: 13 amino acids
(B) TYPE: amino acid
(D) TOPOLOGY: unknown

(ii) MOLECULE TYPE: peptide

(v) FRAGMENT TYPE: internal

(vi) ORIGINAL SOURCE:
(A) ORGANISM: Homo sapiens

(xi) SEQUENCE DESCRIPTION: SEQ ID NO: 2:

Ala Gly Arg Pro Gly Arg Arg Gly Arg Pro Gly Leu Lys
1               5                   10

(2) INFORMATION FOR SEQ ID NO: 3:

(i) SEQUENCE CHARACTERISTICS:
(A) LENGTH: 17 amino acids
(B) TYPE: amino acid
(D) TOPOLOGY: unknown

(ii) MOLECULE TYPE: peptide

(v) FRAGMENT TYPE: internal

(vi) ORIGINAL SOURCE:
(A) ORGANISM: Homo sapiens

(xi) SEQUENCE DESCRIPTION: SEQ ID NO: 3:
Gly Ile Lys Gly Thr Lys Gly Ser Pro Gly Asn Ile Lys Asp Gln Pro
1               5                   10                  15
Arg

(2) INFORMATION FOR SEQ ID NO: 4:

(i) SEQUENCE CHARACTERISTICS:
(A) LENGTH: 20 amino acids
(B) TYPE: amino acid
(D) TOPOLOGY: unknown

(ii) MOLECULE TYPE: peptide

(v) FRAGMENT TYPE: internal

(xi) SEQUENCE DESCRIPTION: SEQ ID NO: 4:
Gly Leu Phe Glu Ala Leu Leu Glu Leu Leu Glu Ser Leu Trp Glu Leu
1               5                   10                  15
Leu Leu Glu Ala
            20

(2) INFORMATION FOR SEQ ID NO: 5:

(i) SEQUENCE CHARACTERISTICS:
(A) LENGTH: 13 amino acids
(B) TYPE: amino acid
(D) TOPOLOGY: unknown

(ii) MOLECULE TYPE: peptide

(v) FRAGMENT TYPE: internal

(xi) SEQUENCE DESCRIPTION: SEQ ID NO: 5:

Tyr Lys Ala Lys Lys Lys Lys Lys Lys Lys Lys Trp Lys
1               5                   10

(2) INFORMATION FOR SEQ ID NO: 6:

(i) SEQUENCE CHARACTERISTICS:
(A) LENGTH: 30 amino acids
(B) TYPE: amino acid
(D) TOPOLOGY: unknown

(ii) MOLECULE TYPE: peptide
(v) FRAGMENT TYPE: internal

(vi) ORIGINAL SOURCE:
(A) ORGANISM: Mus musculus

(xi) SEQUENCE DESCRIPTION: SEQ ID NO: 6:

Val Ala Tyr Ile Ser Arg Gly Gly Val Ser Thr Tyr Tyr Ser Asp Thr
1               5                   10                  15

Val Lys Gly Arg Phe Thr Arg Gln Lys Tyr Asn Lys Arg Ala
            20                  25                  30

### Annex to the application documents - subsequently filed sequences listing

SEQUENCE LISTING

<110> TRANSGENE SA

<120> Antitumoral complexes

<130> Antitumoral complexes

<140>
<141>

<160> 6

<170> PatentIn Ver. 2.1

<210> 1
<211> 23
<212> PRT
<213> Homo sapiens

<400> 1
Cys Arg Ala Pro Asp Gly Lys Lys Gly Glu Ala Gly Arg Pro Gly Arg
  1               5                  10                  15

Arg Gly Arg Pro Gly Leu Lys
             20

<210> 2
<211> 13
<212> PRT
<213> Homo sapiens

<400> 2
Ala Gly Arg Pro Gly Arg Arg Gly Arg Pro Gly Leu Lys
  1               5                  10

<210> 3
<211> 17
<212> PRT
<213> Homo sapiens

<400> 3
Gly Ile Lys Gly Thr Lys Gly Ser Pro Gly Asn Ile Lys Asp Gln Pro
  1               5                  10                  15

Arg

<210> 4
<211> 20
<212> PRT
<213> Homo sapiens

<400> 4
Gly Leu Phe Glu Ala Leu Leu Glu Leu Leu Glu Ser Leu Trp Glu Leu
  1               5                  10                  15

Leu Leu Glu Ala
            20

<210> 5
<211> 13
<212> PRT
<213> Homo sapiens

<400> 5
Tyr Lys Ala Lys Lys Lys Lys Lys Lys Lys Lys Trp Lys
 1               5                   10

<210> 6
<211> 30
<212> PRT
<213> Homo sapiens

<400> 6
Val Ala Tyr Ile Ser Arg Gly Gly Val Ser Thr Tyr Tyr Ser Asp Thr
 1               5                   10                  15

Val Lys Gly Arg Phe Thr Arg Gln Lys Tyr Asn Lys Arg Ala
            20                  25                  30